# FASCICULE DE BREVET EUROPEEN

(11) **EP 3 716 918 B1**
(45) Date de publication et mention de la délivrance du brevet: **09.02.2022**
(21) Numéro de dépôt: 18833260.5
(22) Date de dépôt: 27.11.2018
(51) Int. Cl.: A61F 2/40

(54) **IMPLANT GLÉNOÏDIEN POUR PROTHÈSE D'ÉPAULE**
SCHULTERGELENKIMPLANTAT FÜR SCHULTERPROTHESE
GLENOIDAL IMPLANT FOR SHOULDER PROSTHESIS

(30) Priorité: 28.11.2017 FR 1761298
(43) Date de publication de la demande: 07.10.2020
(73) Titulaire: Shoulder Friends Institute, 75008 Paris (FR)
(72) Inventeur: AUDEBERT, Stéphane, 59268 Blecourt (FR); BARTH, Johannes, 38240 Meylan (FR); CHAROUSSET, Christophe, 75017 Paris (FR); GARRET, Jérôme, 69760 Limonest (FR); GALLINET, David, 25870 Geneuille (FR); GODENECHE, Arnaud, 69450 Saint Cyr Au Mont D'Or (FR); GUERY, Jacques, 58000 Nevers (FR); JOUDET, Thierry, 33500 Libourne (FR); LEFEBVRE, Yves, 67000 Strasbourg (FR)
(74) Mandataire: Germain Maureau
(86) Numéro de dépôt international: PCT/FR2018/052997
(87) Numéro de publication internationale: WO 2019/106280

(56) Documents cités:
- EP-A1- 1 762 201
- WO-A1-2016/110758
- WO-A1-2016/164385
- WO-A2-2017/007565
- FR-A1- 2 937 245

## Description

La présente invention se rapporte à un implant glénoïdien pour prothèse d'épaule.

Un tel implant glénoïdien comprend classiquement un corps articulaire présentant deux faces opposées qui sont une face d'articulation propre à coopérer avec une tête d'articulation d'un implant huméral, et une face d'ancrage de laquelle saille au moins un plot d'ancrage pour un ancrage dans la glène de l'omoplate.

Pour l'ancrage d'un implant glénoïdien dans la glène, il est envisageable de recourir à un ancrage cimentée dans l'os, généralement en présence de structures osseuses moins stables et de moindre qualité que nécessaire comme par exemple du fait d'une ostéoporose, ou bien à un ancrage sans ciment.

Pour un ancrage sans ciment, il est connu d'employer des plots d'ancrage filetés et/ou des plots d'ancrage couverts au moins partiellement d'un revêtement de surface poreux ou rugueux favorisant une ostéo-intégration, comme connu par exemple du document FR 2 971 416. Il est tout de même à noter qu'un ciment peut être employé en complément de tels plots d'ancrage.

L'état de la technique peut également être illustré par l'enseignement du document WO2016/164385 qui décrit un implant glénoïdien pour prothèse d'épaule, comprenant un corps articulaire qui est composé d'une embase frontale (base plate) et d'une platine dorsale (liner) assemblées fixement. Par ailleurs, ce corps articulaire présente deux faces opposées qui sont une face d'articulation sur la platine dorsale propre à coopérer avec une tête d'articulation d'un implant huméral, et une face d'ancrage sur l'embase frontale de laquelle saille un plot d'ancrage principal couvert au moins partiellement d'un revêtement de surface poreux ou rugueux. Ce plot d'ancrage principal est fixé sur l'embase frontale et est pourvu intérieurement d'un trou central qui reçoit intérieurement un plot de centrage (centering feature) intégré à la platine dorsale.

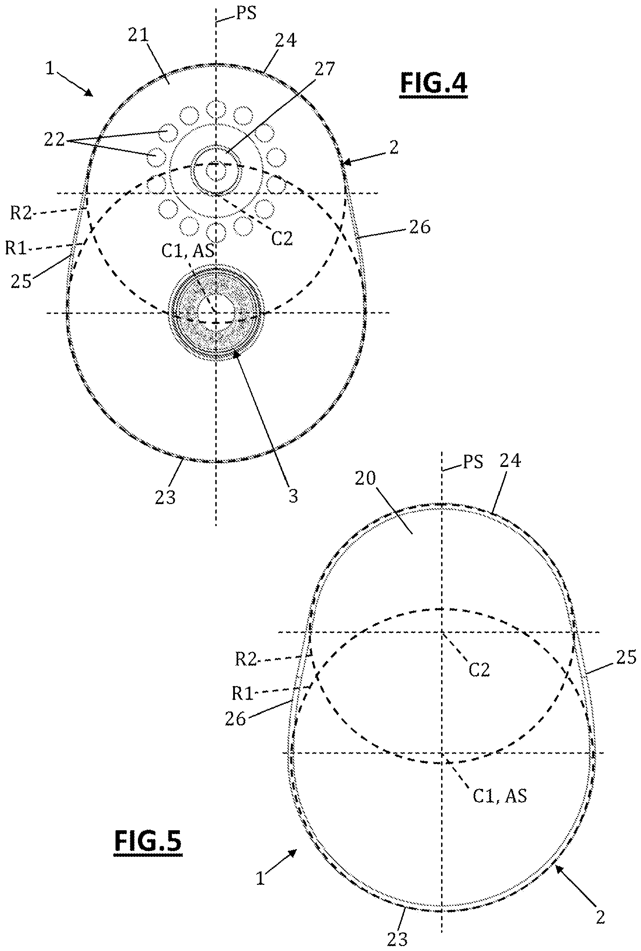

Le document WO2016/110758 divulgue quant à lui un implant glénoïdien pour prothèse d'épaule, comprenant un corps articulaire qui est composé d'une première portion et d'une seconde portion assemblées fixement. Ce corps articulaire présente deux faces opposées qui sont une face d'articulation sur la seconde portion, et une face d'ancrage sur la première portion de laquelle saille un plot d'ancrage principal couvert au moins partiellement d'un revêtement de surface poreux ou rugueux. Ce Par ailleurs, et comme visible aux figures 4 et 5, ce plot d'ancrage principal vient de matière avec la première portion et il est pourvu intérieurement d'un trou central qui est traversant (autrement il débouche sur l'extrémité libre du plot d'ancrage principal) et il est prévu pour recevoir un plot de centrage qui vient de matière avec la seconde portion. Ce plot de centrage reçoit quant à lui intérieurement une vis accessible par la face d'articulation et qui participe au blocage du plot de centrage à l'intérieur du trou central dans le plot d'ancrage principal, et donc à un verrouillage entre la première portion et la seconde portion.

Un plot d'ancrage avec revêtement de surface poreux ou rugueux présente l'avantage que, après implantation dans la glène, les pores sont envahis par les cellules osseuses en croissance pour favoriser une ostéo-intégration de ce plot d'ancrage.

Cependant, si une reprise ou retrait de l'implant glénoïdien est nécessaire, l'ostéo-intégration d'un tel plot d'ancrage contraint le chirurgien à devoir ôter, classiquement au moyen d'une tréfine (ou scie cloche), une grande partie osseuse de la glène pour extraire complètement le plot d'ancrage, avec le risque de nuire à l'ancrage du futur implant glénoïdien qui viendra en remplacement de l'implant glénoïdien retiré. En effet, la préservation du capital osseux est un facteur de succès de la reprise chirurgicale.

La présente invention a notamment pour but de résoudre cet inconvénient, en proposant un implant glénoïdien ayant un plot d'ancrage avec revêtement de surface poreux ou rugueux, et qui permet, lors d'une reprise de l'implant glénoïdien, de guider le chirurgien dans son opération de retrait afin qu'il ôte le minimum d'os dans la glène pour extraire le plot d'ancrage.

A cet effet, elle propose un implant glénoïdien pour prothèse d'épaule, comprenant un corps articulaire présentant deux faces opposées qui sont une face d'articulation propre à coopérer avec une tête d'articulation d'un implant huméral, et une face d'ancrage de laquelle saille au moins un plot d'ancrage pour un ancrage dans la glène dont un plot d'ancrage principal couvert au moins partiellement d'un revêtement de surface poreux ou rugueux favorisant une ostéo-intégration, dans lequel le plot d'ancrage principal est pourvu intérieurement d'un trou central s'étendant selon un axe central de symétrie du plot d'ancrage principal, et dans lequel le plot d'ancrage principal est rapporté fixement sur la face d'ancrage du corps articulaire, et ce plot d'ancrage principal présente deux extrémités opposées qui sont une extrémité proximale solidaire de la face d'ancrage et dans laquelle débouche le trou central, et une extrémité distale bouchée, cet implant glénoïdien étant remarquable en ce que le trou central s'étend sur au moins 80% d'une longueur du plot d'ancrage principal mesurée entre l'extrémité proximale et l'extrémité distale pour permettre un guidage d'une tréfine.

Ainsi, lors d'une opération de reprise de l'implant glénoïdien, le chirurgien pourra, après avoir accédé au trou central du plot d'ancrage principal, engager un guide central d'une tréfine dans ce trou central (le guide central de la tréfine définissant l'axe de symétrie de la découpe circulaire mise en œuvre par la tréfine) et pourra ainsi guider la tréfine pour couper l'os de manière ajustée autour du plot d'ancrage principal, limitant ainsi la dimension de l'orifice de découpe qui restera une fois le plot d'ancrage principal extrait.

Par ailleurs, ce trou central est suffisamment profond (sa profondeur étant au moins égale à 80% de la longueur du plot d'ancrage principal à retirer lors d'une opération de reprise) pour justement permettre de manière précise, stable et complète une telle reprise au moyen de la tréfine engagée dans ce trou central.

Avantageusement, le trou central est vide sur au moins 80% de sa profondeur, et de préférence le trou central est vide sur toute sa profondeur.

Ainsi, ce trou central est vide ou quasiment vide pour favoriser l'insertion et le passage de la tréfine sans qu'un plot ne la gêne.

Selon une possibilité, le trou central présente une entrée sur l'extrémité proximale du plot d'ancrage principal, et cette entrée est bouchée par le corps articulaire.

Ainsi, ce trou central ne débouche pas dans la face d'articulation, ce qui permet de ne pas avoir de débris qui pénètreraient à l'intérieur du trou central et qui nuiraient ensuite au guidage de la tréfine. Par ailleurs, d'une opération de reprise de l'implant glénoïdien, le chirurgien pourra accéder au trou central du plot d'ancrage principal après avoir percé le corps articulaire.

Dans une réalisation particulière, le plot d'ancrage principal est rapporté fixement sur la face d'ancrage du corps articulaire, et ce plot d'ancrage principal présente deux extrémités opposées qui sont une extrémité proximale solidaire de la face d'ancrage et dans laquelle débouche le trou central, et une extrémité distale bouchée, ledit trou central étant borgne.

Selon une caractéristique, le plot d'ancrage principal présente une gorge périphérique au niveau de l'extrémité proximale, et le plot d'ancrage principal est rapporté fixement sur la face d'ancrage du corps articulaire par surmoulage du corps articulaire dans la gorge périphérique.

Selon une autre caractéristique, le trou central présente une entrée conique ou chanfreinée sur l'extrémité proximale du plot d'ancrage principal., afin de guider l'introduction du guide central de la tréfine à l'intérieur du trou central.

Avantageusement, la face d'ancrage du corps articulaire présente une pluralité de renfoncements formant une macrostructure destinée à favoriser une mise en pression d'une couche de ciment qui serait insérée entre la face d'ancrage et l'os.

Selon une possibilité de l'invention, un plot d'ancrage secondaire saille de la face d'ancrage du corps articulaire.

Selon une autre possibilité de l'invention, ce plot d'ancrage secondaire est un plot conique venant de matière avec le corps articulaire.

Dans une réalisation particulière, le corps articulaire présente un plan de symétrie et présente des bords reliant la face d'articulation et la face d'ancrage et comprenant :
- un bord inférieur épousant un cercle principal ayant un diamètre principal prédéfini et un centre principal placé sur le plan de symétrie,
- un bord supérieur épousant un cercle secondaire ayant un diamètre secondaire prédéfini inférieur au diamètre principal et un centre secondaire placé sur le plan de symétrie et décalé par rapport au centre principal ;
- deux bords latéraux joignant le bord inférieur et le bord supérieur de part et d'autre du plan de symétrie ;
où l'axe central de symétrie du plot d'ancrage principal intersecte le centre principal.

Selon une variante, le corps articulaire est un corps monobloc.

L'invention se rapporte également à une prothèse d'épaule comprenant un implant glénoïdien conforme à l'invention décrite ci-dessus, et comprenant en outre un implant huméral prévu pour un ancrage sur un humérus et comprenant une tête d'articulation conformée pour être articulée sur la face d'articulation du corps articulaire de l'implant glénoïdien.

D'autres caractéristiques et avantages de la présente invention apparaîtront à la lecture de la description détaillée ci-après, d'un exemple de mise en œuvre non limitatif, faite en référence aux figures annexées dans lesquelles :
- les figures 1 à 3 sont des vues schématiques en perspective d'un implant glénoïdien conforme à l'invention, selon trois angles de vue distincts ;
- les figures 4, 5 et 6 sont des vues schématiques de l'implant glénoïdien des figures 1 à 3, respectivement de dos, de face et de côté ;
- la figure 7 est une vue en coupe de l'implant glénoïdien des figures 1 à 3, selon un plan de coupe correspondant au plan de symétrie ; et
- la figure 8 est une vue schématique en perspective du plot d'ancrage principal seul.

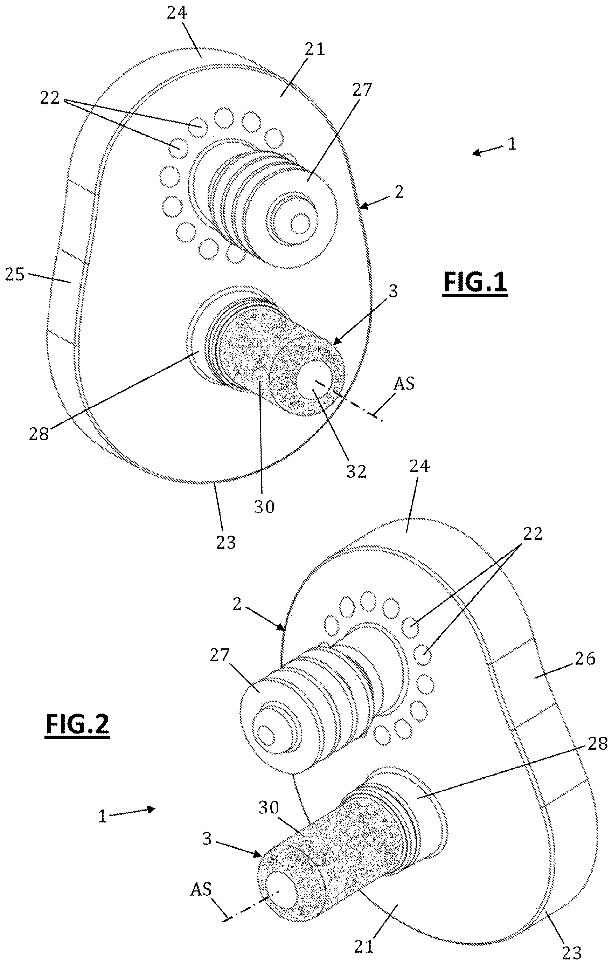

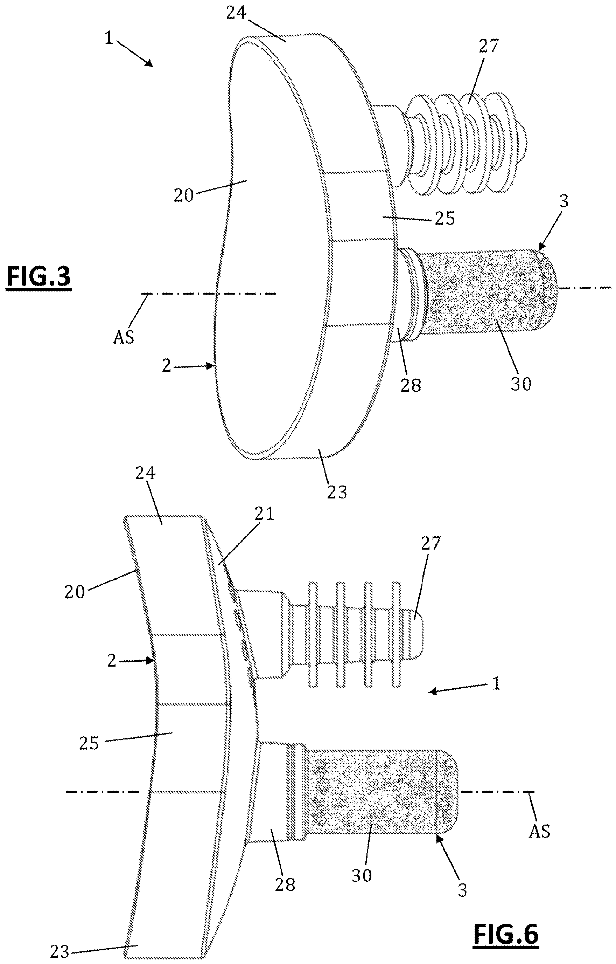

En référence aux figures 1 à 6, un implant glénoïdien 1, conforme à un mode de réalisation de l'invention, comprend un corps articulaire 2 présentant deux faces 20, 21 opposées qui sont une face d'articulation 20 propre à coopérer avec une tête d'articulation (non illustrée) d'un implant huméral, et une face d'ancrage 21 prévue pour un ancrage dans la glène de l'omoplate.

Ce corps articulaire 2 est un corps monobloc réalisé dans un matériau plastique ou polymérique, comme par exemple en polyéthylène, et notamment en polyéthylène haute densité ou de masse molaire ultra élevée (UHMWPE) ou en polyéthylène réticulé (XPE).

Comme visible sur les figures 1, 2 et 4, la face d'ancrage 21 du corps articulaire présente une pluralité de renfoncements 22 formant une macrostructure destinée à favoriser une mise en compression d'une couche de ciment qui serait insérée entre la face d'ancrage 21 et l'os ; ces renfoncements 22 pouvant se présenter sous la forme de renfoncements en pointe de foret à titre d'exemple non limitatif.

Comme visible sur les figures 4 et 5, le corps articulaire 2 présente un plan de symétrie PS et présente des bords 23, 24, 25, 26 reliant la face d'articulation 20 et la face d'ancrage 21, ces bords 23, 24, 25, 26 formant le pourtour périphérique du corps articulaire 2 et définissant l'épaisseur du corps articulaire 2.

Ces bords 23, 24, 25, 26 comprennent :
- un bord inférieur 23 épousant un cercle principal R1 ayant un diamètre principal D1 prédéfini et un centre principal C1 placé sur le plan de symétrie PS;
- un bord supérieur 24 épousant un cercle secondaire R2 ayant un diamètre secondaire D2 prédéfini inférieur au diamètre principal D1, et un centre secondaire C2 placé sur le plan de symétrie PS et décalé par rapport au centre principal C1 ;
- deux bords latéraux 25, 26 joignant le bord inférieur 23 et le bord supérieur 24 de part et d'autre du plan de symétrie PS.

En outre, le corps articulaire 2 comprend un plot d'ancrage secondaire 27 qui saille de la face d'ancrage 21 et qui vient de matière avec le corps articulaire 2. Dans l'exemple illustré sur les figures, ce plot d'ancrage secondaire 27 est un plot conique muni d'ailettes cylindriques espacées les unes des autres. Dans une variante non illustrée, le plot d'ancrage secondaire 27 peut être un plot conique et lisse.

Ce plot d'ancrage secondaire 27, situé dans le plan de symétrie PS, est entouré par le cercle secondaire R2 et est plus précisément placé entre le centre secondaire C2 et le sommet du bord supérieur 24 qui correspond au point du bord supérieur 24 situé dans le plan de symétrie PS.

Dans l'exemple illustré sur les figures, les renfoncements 22 précités sont disposés en périphérie du plot d'ancrage secondaire 27.

L'implant glénoïdien 1 comprend en outre un plot d'ancrage principal 3 qui saille de la face d'ancrage 21 et qui est rapporté fixement sur cette face d'ancrage 21, où ce plot d'ancrage principal 3 est couvert au moins partiellement d'un revêtement de surface poreux ou rugueux 30 favorisant une ostéo-intégration.

Ce plot d'ancrage principal 3 est un plot de révolution centré sur un axe central de symétrie AS qui intersecte le centre principal C1 défini ci-dessus.

Ce plot d'ancrage principal 3 présente deux extrémités opposées qui sont une extrémité proximale 31 solidaire de la face d'ancrage 21 et une extrémité distale 32 libre.

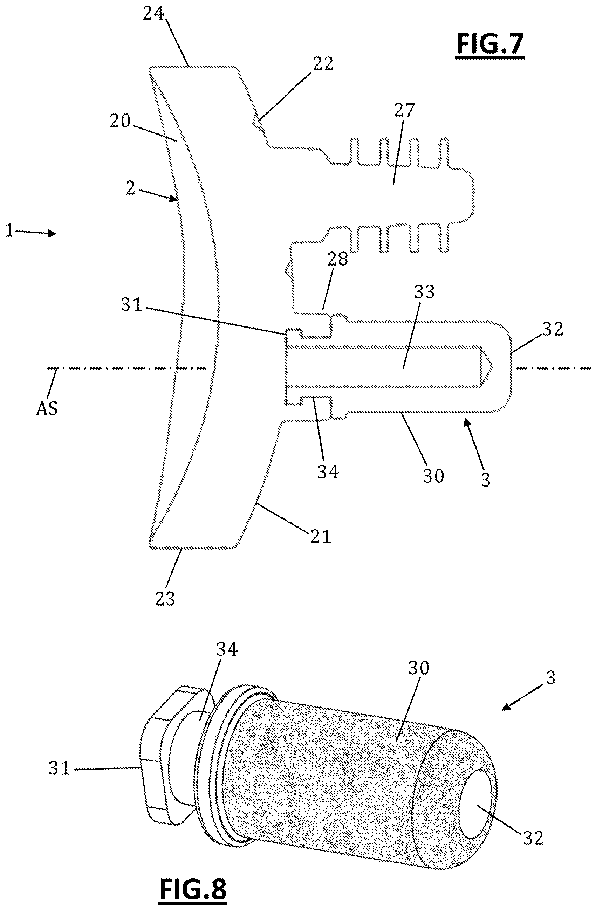

Comme illustré sur la figure 7, ce plot d'ancrage principal 3 est pourvu intérieurement d'un trou central 33 borgne qui débouche dans l'extrémité proximale 31, de sorte que l'extrémité distale 32 est bouchée. Ce trou central 33 prévu pour permettre un guidage d'une tréfine, comme expliqué précédemment.

Ce trou central 33 s'étend sur au moins 80% d'une longueur du plot d'ancrage principal 3 mesurée entre l'extrémité proximale 31 et l'extrémité distale 32. Ce trou central 33 présente donc une profondeur mesurée entre l'extrémité proximale 31 et le fond du trou central 33, et cette profondeur est supérieure ou égale à 0,8 fois la longueur du plot d'ancrage principal 3.

Par ailleurs, et comme visible sur la figure 7, le trou central 33 est vide sur au moins 80% de sa profondeur et même sur toute sa profondeur. Autrement dit, aucun élément externe au trou central 33 n'est introduit à l'intérieur du trou central 33 qui est inoccupé.

Ce trou central 33 présente une entrée sur l'extrémité proximale 31 du plot d'ancrage principal 3, où cette entrée peut être conique ou chanfreinée pour faciliter ultérieurement l'insertion de la tréfine, et par ailleurs cette entrée est bouchée par le corps articulaire 2 en étant noyée à l'intérieur du corps articulaire 2. Ainsi, ce trou central 33 ne débouche pas dans la face d'articulation 20, autrement dit ce trou central n'est pas accessible du côté de la face d'articulation 20, et il est donc nécessaire de percer le corps articulaire pour accéder à l'entrée du trou central 33 pour la tréfine.

Le plot d'ancrage principal 3 présente une gorge périphérique 34 au niveau de l'extrémité proximale 31, et le plot d'ancrage principal 3 est rapporté fixement sur la face d'ancrage 21 par surmoulage du corps articulaire 2 dans la gorge périphérique 34. Autrement dit, le corps articulaire 2 présente, sur sa face d'ancrage 21, un bossage 28 qui surmoule la gorge périphérique 34 du plot d'ancrage principal 3 ; ce bossage 28 faisant partie intégrante du corps articulaire 2.

Le revêtement de surface poreux ou rugueux 30 s'étend sur toute la périphérie du plot d'ancrage principal 3, entre la gorge périphérique 34 et l'extrémité distale 32.

Le revêtement de surface poreux ou rugueux 30 peut par exemple comprendre une couche de titane poreux ou rugueux ou d'un alliage de titane poreux ou rugueux, et éventuellement comprendre en outre une couche de phosphate de calcium, tel que l'hydroxyapatite de calcium.

L'implant glénoïdien 1 peut être utilisé sans ciment avec un ancrage dans la glène qui repose alors uniquement sur l'efficacité du plot d'ancrage principal 3 et de la réintégration de l'os dans son revêtement de surface poreux ou rugueux 30.

L'implant glénoïdien 1 peut aussi être partiellement cimenté, en employant une couche de ciment entre la face d'ancrage 22 et l'os, sans que cette couche de ciment ne vienne dépasser sur le revêtement de surface poreux ou rugueux 30. Dans le cas d'une telle utilisation avec ciment, les renfoncements 22 permettront une mise en compression du ciment, ce qui améliorera sa tenue.

Il est à noter que le plot d'ancrage secondaire 27 a pour fonction principale d'assurer un blocage en rotation du corps articulaire 2 sur la glène.

Bien entendu l'exemple de mise en œuvre évoqué ci-dessus ne présente aucun caractère limitatif et d'autres améliorations et détails peuvent être apportés à l'implant glénoïdien selon l'invention, sans pour autant sortir du cadre de l'invention où d'autres formes ou nombres de plots d'ancrage secondaires peuvent par exemple être réalisées.

## Revendications

1. Implant glénoïdien (1) pour prothèse d'épaule, comprenant un corps articulaire (2) présentant deux faces (20, 21) opposées qui sont une face d'articulation (20) propre à coopérer avec une tête d'articulation d'un implant huméral, et une face d'ancrage (21) de laquelle saille au moins un plot d'ancrage (3 ; 27) pour un ancrage dans la glène dont un plot d'ancrage principal (3) couvert au moins partiellement d'un revêtement de surface poreux ou rugueux (30) favorisant une ostéo-intégration, dans lequel ledit plot d'ancrage principal (3) est pourvu intérieurement d'un trou central (33) s'étendant selon un axe central de symétrie (AS) dudit plot d'ancrage principal (3), et dans lequel le plot d'ancrage principal (3) est rapporté fixement sur la face d'ancrage (21) du corps articulaire (2), et ledit plot d'ancrage principal (3) présente deux extrémités (31, 32) opposées qui sont une extrémité proximale (31) solidaire de la face d'ancrage (21) et dans laquelle débouche le trou central (33), et une extrémité distale (32) bouchée, ledit trou central (33) étant borgne, ledit implant glénoïdien (1) étant **caractérisé en ce que** le trou central (33) s'étend sur au moins 80% d'une longueur du plot d'ancrage principal (3) mesurée entre l'extrémité proximale (31) et l'extrémité distale (32) pour permettre un guidage d'une tréfine.

2. Implant glénoïdien (1) selon la revendication 1, dans lequel le trou central (33) est vide sur au moins 80% de sa profondeur.

3. Implant glénoïdien (1) selon la revendication 2, dans lequel le trou central (33) est vide sur toute sa profondeur.

4. Implant glénoïdien (1) selon l'une quelconque des revendications 1 à 3, dans lequel le trou central (33) présente une entrée sur l'extrémité proximale (31) du plot d'ancrage principal (3), ladite entrée étant bouchée par le corps articulaire (2).

5. Implant glénoïdien (1) selon l'une quelconque des revendications 1 à 4, dans lequel le plot d'ancrage principal (3) présente une gorge périphérique (34) au niveau de l'extrémité proximale (31), et le plot d'ancrage principal (3) est rapporté fixement sur la face d'ancrage (21) du corps articulaire (2) par surmoulage du corps articulaire (2) dans la gorge périphérique (34).

6. Implant glénoïdien (1) selon l'une quelconque des revendications 1 à 5, dans lequel le trou central (33) présente une entrée conique ou chanfreinée sur l'extrémité proximale (31) du plot d'ancrage principal (3).

7. Implant glénoïdien (1) selon l'une quelconque des revendications 1 à 6, dans lequel la face d'ancrage (21) du corps articulaire (2) présente une pluralité de renfoncements (22) formant une macrostructure destinée à favoriser une mise en pression d'une couche de ciment qui serait insérée entre la face d'ancrage et l'os.

8. Implant glénoïdien (1) selon l'une quelconque des revendications 1 à 7, dans lequel un plot d'ancrage secondaire (27) saille de la face d'ancrage (21) du corps articulaire (2).

9. Implant glénoïdien (1) selon la revendication 8, dans lequel le plot d'ancrage secondaire (27) est un plot conique venant de matière avec le corps articulaire (2).

10. Implant glénoïdien (1) selon l'une quelconque des revendications 1 à 9, dans lequel le corps articulaire (2) présente un plan de symétrie (PS) et présente des bords (23, 24, 25, 26) reliant la face d'articulation (20) et la face d'ancrage (21) et comprenant :
- un bord inférieur (23) épousant un cercle principal (R1) ayant un diamètre principal (D1) prédéfini et un centre principal (C1) placé sur le plan de symétrie (PS),
- un bord supérieur (24) épousant un cercle secondaire (R2) ayant un diamètre secondaire (D2) prédéfini inférieur au diamètre principal (D1) et un centre secondaire (C2) placé sur le plan de symétrie (PS) et décalé par rapport au centre principal (C1) ;
- deux bords latéraux (25, 26) joignant le bord inférieur (23) et le bord supérieur (24) de part et d'autre du plan de symétrie (PS) ;
où l'axe central de symétrie (AS) du plot d'ancrage principal (3) intersecte le centre principal (C1).

11. Implant glénoïdien (1) selon l'une quelconque des revendications 1 à 10, dans lequel le corps articulaire (2) est un corps monobloc.

12. Prothèse d'épaule comprenant un implant glénoïdien (1) conforme à l'une quelconque des revendications 1 à 11, et comprenant en outre un implant huméral prévu pour un ancrage sur un humérus et comprenant une tête d'articulation conformée pour être articulée sur la face d'articulation (20) du corps articulaire (2) de l'implant glénoïdien (1).

## Patentansprüche

1. Schultergelenkimplantat (1) für Schulterprothese, umfassend einen Gelenkkörper (2), der zwei gegenüberliegende Flächen (20, 21) aufweist, die eine Gelenkfläche (20), die imstande ist, mit einem Gelenkkopf eines Humerusimplantats zusammenzuwirken, und eine Verankerungsfläche (21) sind, aus der mindestens ein Verankerungsstück (3; 27) für eine Verankerung in der Gelenkpfanne herausragt, davon ein Haupt-Verankerungsstück (3), das mindestens teilweise mit einer porösen oder rauen Oberflächenbeschichtung (30) bedeckt ist, die eine Integration in den Knochen fördert, wobei das Haupt-Verankerungsstück (3) innen mit einem zentralen Loch (33) versehen ist, das sich gemäß einer zentralen Symmetrieachse (AS) des Haupt-Verankerungsstücks (3) erstreckt, und wobei das Haupt-Verankerungsstück (3) auf der Verankerungsfläche (21) des Gelenkkörpers (2) fest angebracht ist, und wobei das Haupt-Verankerungsstück (3) zwei gegenüberliegende Enden (31, 32) aufweist, die ein proximales Ende (31), das mit der Verankerungsfläche (21) fest verbunden ist und in das das zentrale Loch (33) ausmündet, und ein verschlossenes distales Ende (32) sind, wobei das zentrale Loch (33) ein Sackloch ist, wobei das Schultergelenkimplantat (1) **dadurch gekennzeichnet ist, dass** sich das zentrale Loch (33) über mindestens 80 % einer Länge des Haupt-Verankerungsstücks (3) erstreckt, gemessen zwischen dem proximalen Ende (31) und dem distalen Ende (32), um die Führung eines Trepans zu erlauben.

2. Schultergelenkimplantat (1) nach Anspruch 1, wobei das zentrale Loch (33) über mindestens 80 % seiner Tiefe leer ist.

3. Schultergelenkimplantat (1) nach Anspruch 2, wobei das zentrale Loch (33) über seine gesamte Tiefe leer ist.

4. Schultergelenkimplantat (1) nach einem der Ansprüche 1 bis 3, wobei das zentrale Loch (33) einen Eingang auf dem proximalen Ende (31) des Haupt-Verankerungsstücks (3) aufweist, wobei der Eingang durch den Gelenkkörper (2) verschlossen ist.

5. Schultergelenkimplantat (1) nach einem der Ansprüche 1 bis 4, wobei das Haupt-Verankerungsstück (3) eine periphere Nut (34) im Bereich des proximalen Endes (31) aufweist und das Haupt-Verankerungsstück (3) auf der Verankerungsfläche (21) des Gelenkkörpers (2) durch Overmoulding des Gelenkkörpers (2) in der peripheren Nut (34) fest angebracht ist.

6. Schultergelenkimplantat (1) nach einem der Ansprüche 1 bis 5, wobei das zentrale Loch (33) einen konischen oder abgeschrägten Eingang auf dem proximalen Ende (31) des Haupt-Verankerungsstücks (3) aufweist.

7. Schultergelenkimplantat (1) nach einem der Ansprüche 1 bis 6, wobei die Verankerungsfläche (21) des Gelenkkörpers (2) eine Vielzahl von Nischen (22) aufweist, die eine Makrostruktur bilden, die bestimmt ist, eine Unterdrucksetzung einer Zementschicht zu fördern, die zwischen der Verankerungsfläche und dem Knochen eingesetzt ist.

8. Schultergelenkimplantat (1) nach einem der Ansprüche 1 bis 7, wobei ein sekundäres Verankerungsstück (27) aus der Verankerungsfläche (21) des Gelenkkörpers (2) herausragt.

9. Schultergelenkimplantat (1) nach Anspruch 8, wobei das sekundäre Verankerungsstück (27) ein konisches Stück ist, das mit dem Gelenkkörper (2) materialeinheitlich ist.

10. Schultergelenkimplantat (1) nach einem der Ansprüche 1 bis 9, wobei der Gelenkkörper (2) eine Symmetrieebene (PS) aufweist und Ränder (23, 24, 25, 26) aufweist, die die Gelenkfläche (20) und die Verankerungsfläche (21) verbinden und umfassen:
- einen unteren Rand (23), der sich an einen Hauptkreis (R1) mit einem vorher festgelegten Hauptdurchmesser (D1) und einem Hauptzentrum (C1) schmiegt, das auf der Symmetrieebene (PS) platziert ist,
- einen oberen Rand (24), der sich an einen sekundären Kreis (R2) mit einem vorher festgelegten sekundären Durchmesser (D2), der kleiner als der Hauptdurchmesser (D1) ist, und einem sekundären Zentrum (C2) schmiegt, das auf der Symmetrieebene (PS) platziert und im Verhältnis zum Hauptzentrum (C1) versetzt ist;
- zwei seitliche Ränder (25, 26), die den unteren Rand (23) und den oberen Rand (24) beiderseits der Symmetrieebene (PS) verbinden;
wobei die zentrale Symmetrieachse (AS) des Haupt-Verankerungsstücks (3) das Hauptzentrum (C1) schneidet.

11. Schultergelenkimplantat (1) nach einem der Ansprüche 1 bis 10, wobei der Gelenkkörper (2) ein einteiliger Körper ist.

12. Schulterprothese, die ein Schultergelenkimplantat (1) nach einem der Ansprüche 1 bis 11 umfasst und ferner ein Humerusimplantat umfasst, das für eine Verankerung auf einem Humerus vorgesehen ist und einen Gelenkkopf umfasst, der ausgebildet ist, um auf der Gelenkfläche (20) des Gelenkkörpers (2) des Schultergelenkimplantats (1) angelenkt zu sein.

## Claims

1. A glenoidal implant (1) for a shoulder prosthesis, comprising an articular body (2) having two opposite faces (20, 21) which are an articulation face (20) suitable for cooperating with an articulation head of a humeral implant, and an anchoring face (21) from which at least one anchoring stud (3 ; 27) protrudes for an anchoring in the glenoid cavity including a main anchoring stud (3) at least partially covered with a porous or rough surface coating (30) promoting an osteo integration, in which said main anchoring stud (3) is provided internally with a central hole (33) extending along a central axis of symmetry (AS) of said main anchoring stud (3), and in which the main anchoring stud (3) is fixedly mounted on the anchoring face (21) of the articular body (2), and said main anchoring stud (3) has two opposite ends (31, 32) which are a proximal end (31) secured to the anchoring face (21) and in which the central hole (33) opens, and a stoppered distal end (32), said central hole (33) being blind, said glenoidal implant (1) being **characterized in that** the central hole (33) extends over at least 80% of a length of the main anchoring stud (3) measured between the proximal end (31) and the distal end (32) to allow guiding a trephine.

2. The glenoidal implant (1) according to claim 1, wherein the central hole (33) is empty on at least 80% of its depth.

3. The glenoidal implant (1) according to claim 2, wherein the central hole (33) is empty over its entire depth.

4. The glenoidal implant (1) according to any one of claims 1 to 3, wherein the central hole (33) has an entrance on the proximal end (31) of the main anchoring stud (3), said entrance being stoppered by the articular body (2).

5. The glenoidal implant (1) according to any one of claims 1 to 4, wherein the main anchoring stud (3) has a peripheral groove (34) at the proximal end (31), and the main anchoring stud (3) is fixedly mounted on the anchoring face (21) of the articular body (2) by overmolding the articular body (2) in the peripheral groove (34).

6. The glenoidal implant (1) according to any one of claims 1 to 5, wherein the central hole (33) has a conical or chamfered entrance on the proximal end (31) of the main anchoring stud (3).

7. The glenoidal implant (1) according to any one of claims 1 to 6, wherein the anchoring face (21) of the articular body (2) has a plurality of recesses (22) forming a macrostructure intended to promote a pressurization of a cement layer which would be inserted between the anchoring face and the bone.

8. The glenoidal implant (1) according to any one of claims 1 to 7, wherein a secondary anchoring stud (27) protrudes from the anchoring face (21) of the articular body (2).

9. The glenoidal implant (1) according to claim 8, wherein the secondary anchoring stud (27) is a conical stud being integral with the articular body (2).

10. The glenoidal implant (1) according to any one of claims 1 to 9 , wherein the articular body (2) has a plane of symmetry (PS) and has edges (23, 24, 25, 26) connecting the articulation face (20) and the anchoring face (21) and comprising:
- a lower edge (23) matching a main circle (R1) having a predefined main diameter (D1) and a main center (C1) placed on the plane of symmetry (PS),
- an upper edge (24) matching a secondary circle (R2) having a predefined secondary diameter (D2) less than the main diameter (D1) and a secondary center (C2) placed on the plane of symmetry (PS) and offset relative to the main center (C1);
- two lateral edges (25, 26) joining the lower edge (23) and the upper edge (24) on either side of the plane of symmetry (PS);
wherein the central axis of symmetry (AS) of the main anchoring stud (3) intersects the main center (C1).

11. The glenoidal implant (1) according to any one of claims 1 to 10, wherein the articular body (2) is a one-piece body.

12. A shoulder prosthesis comprising a glenoidal implant (1) according to any one of claims 1 to 11, and further comprising a humeral implant provided for an anchoring on a humerus and comprising an articulation head shaped to be joint on the articulation face (20) of the articular body (2) of the glenoidal implant (1) .
